# EUROPEAN PATENT SPECIFICATION

(11) **EP 1 178 035 B1**
(45) Date of publication and mention of the grant of the patent: **30.07.2008**
(21) Application number: 01420177.6
(22) Date of filing: 02.08.2001
(51) Int. Cl.: C07C 251/24, C07D 277/28, C07D 207/32, C07D 213/53, C07D 307/70, A01N 35/10, A01N 43/78, A01N 43/36, A01N 43/08, A01N 43/40

(54) **Fungicidal phenylimine derivatives**
Fungizide Phenyliminderivate
Dérivés de phénylimines fongicides

(30) Priority: 04.08.2000 EP 00116819
(43) Date of publication of application: 06.02.2002
(73) Proprietor: Bayer CropScience S.A., 69009 Lyon (FR)
(72) Inventor: Gerusz, Vincent, 69009 Lyon (FR); Mansfield, Darren James, 69004 Lyon (FR); Perez, Joseph, 69009 Lyon (FR); Vors, Jean-Pierre, 69009 Lyon (FR)
(74) Representative: Nowak, Alexander

(56) References cited:
- EP-A- 0 563 384
- WO-A-00/46184
- WO-A-99/21837
- DE-A- 19 623 744
- GB-A- 1 413 513
- US-A- 4 059 590
- US-A- 4 389 236
- US-A- 4 659 360
- US-A- 5 468 857
- PATENT ABSTRACTS OF JAPAN vol. 009, no. 283 (C-313), 9 November 1985 (1985-11-09) -& JP 60 126267 A (CHUGAI SEIYAKU KK), 5 July 1985 (1985-07-05)
- DATABASE WPI Section Ch, Week 197845 Derwent Publications Ltd., London, GB; Class C02, AN 1978-80909A XP002148259 -& JP 53 113024 A (NIPPON SODA CO), 3 October 1978 (1978-10-03)
- PATENT ABSTRACTS OF JAPAN vol. 1999, no. 12, 29 October 1999 (1999-10-29) -& JP 11 180964 A (MITSUBISHI CHEMICAL CORP;NIPPON SHOKUBUTSU CHOSETSUZAI KENKYU KYOKAI), 6 July 1999 (1999-07-06)
- GUPTA S ET AL.: "Studies on potential pesticides: Part XI. Synthesis of various thiourea and Schiff base derivatives of bis(4-aminophenyl)methane and their biological activities" INDIAN JOURNAL OF CHEMISTRY, SECTION B: ORGANIC, INCL. MEDICINAL., vol. 18, no. 4, 1979, pages 381-382, XP000940603 PUBLICATIONS & INFORMATIONS DIRECTORATE, NEW DELHI., IN ISSN: 0019-5103
- DATABASE CAPLUS [Online] CAS; XP002148258 & NIPPON NOYAKU GAKKAISHI, vol. 7, no. 8, 1982, pages 373-376,

## Description

This invention relates to new fungicidal phenylimine derivatives, their process of preparation and the fungicidal compositions containing them.

Many fungicidal compounds are disclosed in the literature (see, for ex., JP 60126267, US5468857, JP 53113024, US 4059590, DE 19623744, US4389236, JP11180964, EP 0563384, WO9921837)

WO 95/22532 relates to substituted phenyltriazolinones claimed as herbicides and discloses *inter alia* a compound of formula A for which there is no characterising data therein.

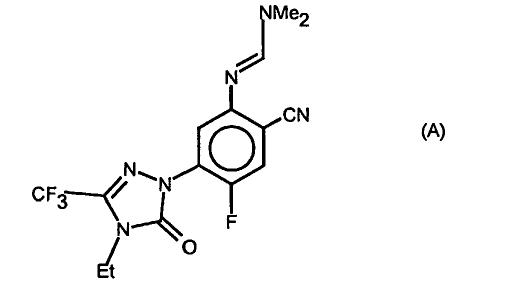

The abstract, composition claim and use claim refer only to the use of such compounds as herbicides and indeed the description supports the invention only with herbicidal activity data. There is a sentence in the specification that states that certain compounds show fungicidal activity, although no fungicidal activity data are provided. No indication is given as to which compounds are fungicidal and there is no suggestion that compound A could be fungicidal.

We have now found that certain phenylimines have fungicidal activity. Therefore, the invention provides the use of a compound of general formula (I) and salts thereof as fungicides : 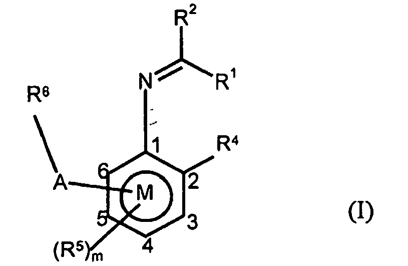 wherein
- R¹ and R², which may be the same or different, are chosen from among alkyl, acyl, cyano, alkoxycarbonyl, aminocarbonyl, alkenyl, alkynyl, carbocyclyl, heterocyclyl, each of which may be substituted, and hydrogen; or
- R² and R¹, together with their interconnecting atoms may form a ring, which may be substituted;
- R⁴ is chosen from among alkyl, alkenyl, alkynyl, carbocyclyl, heterocyclyl, each of which may be substituted; hydroxy; mercapto; azido; nitro; halogen; cyano; acyl; optionally substituted amino; cyanato; thiocyanato; -SF₅; -OR^{a}; -SR^{a} and -Si(R^{a})₃, where R^{a} is alkyl, alkenyl, alkynyl, acyl, carbocyclyl or heterocyclyl, each of which may be substituted;
- m is 0 to 3;
- when present R⁵, which may be the same or different to any other R⁵, is any group defined for R⁴;
- R⁶ is optionally substituted carbo- or heterocyclyl; and
- A is a direct bond, -O-, -S(O)ₙ-, -NR⁹-, -CR⁷=CR⁷-, -C≡C-, -A¹-, -A¹-A¹-, -O-(A¹)ₖ-O-, -O-(A¹)ₖ-, -A³-, -A⁴-, -A¹O-, -A¹S(O)ₙ-, -A²-, -OA²-, -NR⁹A²-, -OA²-A¹-, -OA²-C(R⁷)=C(R⁸)-, -S(O)ₙA¹-, -A¹-A⁴-, -A¹-A⁴-C(R⁸)=N-N=CR⁸-, -A¹-A⁴-C(R⁸)=N-X²-X³-, -A¹-A⁴-A³-, -A¹-A⁴-N(R⁹)-, -A¹-A⁴-X-CH₂-, -A¹-A⁴-A¹-, -A¹-A⁴-CH₂X-, -A¹-A⁴-C(R⁸)=N-X²-X³-X¹-, -A¹-X-C(R⁸)=N-, -A¹-X-C(R⁸)=N-N=CR⁸-, -A¹-X-C(R⁸)=N-N(R⁹)-, -A¹-X-A²-X¹-, -A¹-O-A³-, -A¹-O-C(R⁷)=C(R⁸)-, -A¹-O-N(R⁹)-A²-N(R⁹)-, -A¹-O-N(R⁹)-A²-, -A¹-N(R⁹)-A²-N(R⁹)-, -A¹-N(R⁹)-A²-, -A¹-N(R⁹)-N=C(R⁸)-, -A³-A¹-, -A⁴-A³-, -A²-NR⁹-, -A¹-A²-X¹-, -A¹-A¹-A²-X¹-, -O-A²-N(R⁹)-A²-, -CR⁷=CR⁷-A²-X¹-, -C≡C-A²-X¹-, -N=C(R⁸)-A²-X¹-, -C(R⁸)=N-N=C(R⁸)-, -C(R⁸)=N-N(R⁹)-, -(CH₂)₂-O-N=C(R⁸)- ou -X-A²-N(R⁹)-
where:
- n is 0, 1 or 2,
- k is 1 to 9,
- A¹ is -CHR⁷-,
- A² is -C(=X)-,
- A³ is -C(R⁸)=N-O-,
- A⁴ is -O-N=C(R⁸)-,
- X is O or S,
- X¹ is O, S, NR⁹ or a direct bond,
- X² is O, NR⁹ or a direct bond,
- X³ is hydrogen, -C(=O)-, -SO₂- or a direct bond,
- R⁷, which may be the same or different to any other R⁷, is alkyl, alkenyl, alkynyl, cyano, acyl, hydroxy, alkoxy, haloalkoxy, alkylthio, cycloalkyl or phenyl, each of which may be substituted; or is hydrogen or halogen;
- R⁸, which may be the same or different to any other R⁸, is alkyl, alkenyl, alkynyl, alkoxy, alkylthio, carbo- or hetero-cyclyl, each of which may be substituted; or is hydrogen;
- R⁹, which may be the same or different to any other R⁹, is optionally substituted alkyl, optionally substituted carbo- or hetero-cyclyl, hydrogen or acyl; or two R⁹ groups on A, together with the connecting atoms, form a 5 to 7 membered ring;
where the moiety depicted on the right side of linkage A is attached to R⁶; or -A-R⁶ and R⁵ together with benzene ring M form an optionally substituted fused ring system.

Preferably R¹ is alkyl, alkenyl or alkynyl, each of which may be substituted by alkoxy, haloalkoxy, alkylthio, halogen or optionally substituted phenyl (preferably phenyl optionally substituted by alkyl, haloalkyl, alkoxy, haloalkoxy or alkylthio, each containing 1 to 5 carbon atoms, or halogen), or is hydrogen or cyano. R¹ is especially C₁-C₁₀ alkyl (e.g. methyl) or hydrogen.

Preferably R² is alkyl, acyl, alkoxycarbonyl, aminocarbonyl, alkenyl or alkynyl, each of which may be substituted by alkoxy, haloalkoxy, alkylthio, halogen or optionally substituted phenyl (preferably phenyl, optionally substituted by alkyl, haloalkyl, alkoxy, haloalkoxy or alkylthio, each containing 1 to 5 carbon atoms, or by halogen), or is hydrogen, cyano or alkylcarbonyl. R² is especially C₁-C₁₀ alkyl (e.g. methyl or ethyl) or hydrogen.

Preferably R⁴ is alkyl, alkenyl, or alkynyl, each of which may be substituted by alkoxy, haloalkoxy, alkylthio, halogen or optionally substituted phenyl (preferably phenyl optionally substituted by alkyl, haloalkyl, alkoxy, haloalkoxy or alkylthio, each containing 1 to 5 carbon atoms, or halogen); or is hydroxy; halogen; cyano; acyl (preferably -C(=O)R^{c}, -C(=S)R^{c} or -S(O)ₚR^{c}, where R^{c} is alkyl, haloalkyl, alkoxy, haloalkoxy, alkylthio, amino, monoalkylamino, dialkylamino or phenyl optionally substituted by alkyl, haloalkyl, alkoxy, haloalkoxy or alkylthio; or phenyloxy, phenylthio, carbocyclyl, heterocyclyl); alkoxy; haloalkoxy; or alkylthio. R⁴ is especially C₁-C₁₀ alkyl (e.g. methyl or ethyl) or halogen.

Preferably m is 0 or 1, especially 1.

When present, R⁵ is preferably a group defined for preferred R⁴ above.
R⁵ is especially C₁-C₁₀ alkyl or halogen.

When present, the group R⁵ is preferably attached at the 5 position of ring M.

Preferably A is a direct bond, -O-, -A¹-, -S(O)ₙA¹-, -O(A¹)ₖ-, -S(O)ₙ-, -NR⁹A²-, -A²-, -OA²-, -OA²-A¹-, -NR⁹- or -O(A¹)ₖO-. Particularly A is a direct bond, -O-, -S-, -NR⁹-, -CHR⁷- or -O-CHR⁷-. Especially A is a direct bond or -O-.

When present, R⁹ is alkyl, alkenyl, or alkynyl, each of which may be substituted by alkoxy, haloalkoxy, alkylthio, halogen or optionally substituted phenyl (preferably phenyl optionally substituted by alkyl, haloalkyl, alkoxy, haloalkoxy or alkylthio, each containing 1 to 5 carbon atoms, or halogen); or is hydrogen (R⁹ is especially C₁-C₁₀ alkyl or hydrogen).

When present, R⁷ is alkyl, alkenyl, or alkynyl, each of which may be substituted by alkoxy, haloalkoxy, alkylthio, halogen or optionally substituted phenyl (preferably phenyl optionally substituted by alkyl, haloalkyl, alkoxy, haloalkoxy or alkylthio, each containing 1 to 5 carbon atoms, or by halogen); or is hydroxy; halogen; cyano; acyl; alkoxy; haloalkoxy; alkylthio; or hydrogen (R⁷ is especially C₁-C₁₀ alkyl or hydrogen).

Preferably A is attached to the 4 position of benzene ring M.

Preferably R⁶ is optionally substituted phenyl or optionally substituted aromatic heterocyclyl [preferably thiazolyl, isothiazolyl, thiadiazolyl (particularly 1,2,4-thiadiazolyl), pyridyl or pyrimidinyl].

When substituted, R⁶ may be substituted by one or more substituents, which may be the same or different, and may be selected from the preferred list: alkyl, alkenyl, alkynyl, carbo- or heterocyclyl, each of which may be substituted; hydroxy; mercapto; azido; nitro; halogen; cyano; acyl; optionally substituted amino; cyanato; thiocyanato; -SF₅; -OR^{a}; -SR^{a} and -Si(R^{a})₃, where R^{a} is alkyl, alkenyl, alkynyl, carbocyclyl or heterocyclyl, each of which may be substituted.

A preferred list of substituents on R⁶ is: hydroxy; halogen; cyano; acyl (preferably -C(=O)R^{c}, -C(=S)R^{c} or -S(O)ₚR^{c}, where R^{c} is alkyl, haloalkyl, alkoxy, haloalkoxy, alkylthio, amino, monalkylamino, dialkylamino or phenyl optionally substituted by alkyl, haloalkyl, alkoxy, haloalkoxy or alkylthio; or phenyloxy, phenylthio, carbocyclyl, or heterocyclyl); amino; alkylamino; dialkylamino; alkyl; haloalkyl; R^{a}O-alkyl; acyloxyalkyl; cyano-oxyalkyl; alkoxy; haloalkoxy; alkylthio; carbocyclyl (preferably cyclohexyl or cyclopentyl) optionally substituted by alkyl, haloalkyl, alkoxy, haloalkoxy or alkylthio; and benzyl optionally substituted by alkyl, haloalkyl, alkoxy, haloalkoxy or alkylthio.

In a preferred embodiment, the invention provides the use of a compound of general formula (I) and salts thereof as fungicides wherein:
- R¹ is alkyl, alkenyl or alkynyl, each of which may be substituted by alkoxy, haloalkoxy, alkylthio, halogen or phenyl optionally substituted by alkyl, haloalkyl, alkoxy, haloalkoxy, alkylthio or halogen; or is hydrogen;
- R² is as defined for R¹ in this embodiment, or is acyl, aminocarbonyl or alkylcarbonyl;
- R⁴ is alkyl, alkenyl or alkynyl, each of which may be substituted by alkoxy, haloalkoxy, alkylthio, halogen or phenyl optionally substituted by alkyl, haloalkyl, alkoxy, haloalkoxy, alkylthio or halogen; or is hydroxy; halogen; cyano; acyl (preferably -C(=O)R^{c}, -C(=S)R^{c} or -S(O)ₚR^{c}, where R^{c} is alkyl, haloalkyl, alkoxy, haloalkoxy, alkylthio, amino, monoalkylamino, dialkylamino or phenyl optionally substituted by alkyl, haloalkyl, alkoxy, haloalkoxy or alkylthio; or phenyloxy, phenylthio, carbocyclyl, heterocyclyl);
- m is 0 or 1;
- when present, R⁵ is a group defined for R⁴ in this embodiment;
- A is a direct bond, -O-, -S-, -NR⁹-, -CHR⁷- or -O-CHR⁷-,
wherein when present, R⁹ is alkyl, alkenyl, or alkynyl, each of which may be substituted by alkoxy, haloalkoxy, alkylthio, halogen or phenyl optionally substituted by alkyl, haloalkyl, alkoxy, haloalkoxy, alkylthio, or halogen; or is hydrogen; and R⁷ is a group defined for R⁹ in this embodiment, or is hydroxy; halogen; cyano; acyl; alkoxy; haloalkoxy or alkylthio;
- A is attached to the 4 position of benzene ring M; and
- R⁶ is phenyl or aromatic heterocyclyl, optionally substituted by one or more substituents, which may be the same or different, and may be selected from the list: hydroxy; halogen; cyano; acyl (preferably -C(=O)R^{c}, -C(=S)R^{c} or -S(O)ₚR^{c}, where R^{c} is alkyl, haloalkyl, alkoxy, haloalkoxy, alkylthio, amino, monoalkylamino, dialkylamino or phenyl optionally substituted by alkyl, haloalkyl, alkoxy, haloalkoxy or alkylthio; or phenyloxy, phenylthio, carbocyclyl, heterocyclyl); amino; alkylamino; dialkylamino; alkyl; haloalkyl; R^{a}O-alkyl; acyloxyalkyl; cyano-oxyalkyl; alkoxy; haloalkoxy; alkylthio; carbocyclyl (preferably cyclohexyl or cyclopentyl) optionally substituted by alkyl, haloalkyl, alkoxy, haloalkoxy or alkylthio; and benzyl optionally substituted by alkyl, haloalkyl, alkoxy, haloalkoxy or alkylthio.

Any alkyl group may be straight or branched and is preferably of 1 to 10 carbon atoms, especially 1 to 7 and particularly 1 to 5 carbon atoms.

Any alkenyl or alkynyl group may be straight or branched and is preferably of 2 to 7 carbon atoms and may contain up to 3 double or triple bonds which may be conjugated, for example vinyl, allyl, butadienyl or propargyl.

Any carbocyclyl group may be saturated, unsaturated or aromatic, and contain 3 to 8 ring-atoms. Preferred saturated carbocyclyl groups are cyclopropyl, cyclopentyl or cyclohexyl. Preferred unsaturated carbocyclyl groups contain up to 3 double bonds. A preferred aromatic carbocyclyl group is phenyl. The term carbocylic should be similarly construed. In addition, the term carbocyclyl includes any fused combination of carbocyclyl groups, for example naphthyl, phenanthryl, indanyl and indenyl.

Any heterocyclyl group may be saturated, unsaturated or aromatic, and contain 3 to 7 ring-atoms up to 4 of which may be hetero-atoms such as nitrogen, oxygen and sulphur. Examples of heterocyclyl groups are furyl, thienyl, pyrrolyl, pyrrolinyl, pyrrolidinyl, imidazolyl, dioxolanyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridyl (and pyridyl N-oxide), piperidinyl, dioxanyl, morpholino, dithianyl, thiomorpholino, pyridazinyl, pyrimidinyl, pyrazinyl, piperazinyl, sulpholanyl, tetrazolyl, triazinyl, azepinyl, oxazepinyl, thiazepinyl, diazepinyl and thiazolinyl.

In addition, the term heterocyclyl includes fused heterocyclyl groups, for example benzimidazolyl, benzoxazolyl, imidazopyridinyl, benzoxazinyl, benzothiazinyl, oxazolopyridinyl, benzofuranyl, quinolinyl, quinazolinyl, quinoxalinyl, dihydroquinazolinyl, benzothiazolyl, phthalimido, benzofuranyl, benzodiazepinyl, indolyl and isoindolyl. The term heterocyclic should be similarly construed.

Any alkyl, alkenyl, alkynyl, carbocyclyl or heterocyclyl group, when substituted, may be substituted by one or more substituents, which may be the same or different, and may be selected from the list: hydroxy; mercapto; azido; nitro; halogen; cyano; acyl; alkoxycarbonyl; optionally substituted aminocarbonyl; optionally substituted amino; optionally substituted ammonio; optionally substituted carbocyclyl; optionally substituted heterocyclyl; cyanato; thiocyanato; -SF₅; -OR^{a};-SR^{a}; -SOR^{a}; -SO₂R^{a} and -Si(R^{a})₃, where R^{a} is alkyl, alkenyl, alkynyl, carbocyclyl or heterocyclyl, each of which may be substituted. In the case of any carbocyclyl or heterocyclyl group the list includes additionally: alkyl, alkenyl and alkynyl, each of which may be substituted. Preferred substituents on any alkyl, alkenyl or alkynyl group are alkoxy, haloalkoxy or alkylthio, each containing 1 to 5 carbon atoms; halogen; or optionally substituted phenyl. Preferred substituents on any carbocyclyl or heterocyclyl group are alkyl, haloalkyl, alkoxy, haloalkoxy or alkylthio, each containing 1 to 5 carbon atoms; halogen; or optionally substituted phenyl.

In the case of any alkyl group or any unsaturated ring-carbon in any carbocyclyl or heterocyclyl group the list includes a divalent group such as oxo or imino, which may be substituted by optionally substituted amino, R^{a} or -OR^{a} (where R^{a} is as defined above). Preferred groups are oxo, imino, alkylimino, oximino, alkyloximino or hydrazono.

Any amino group, when substituted and where appropriate, may be substituted by one or two substituents which may be the same or different, selected from the list: optionally substituted alkyl, optionally substituted amino, -OR^{a} (where R^{a} is as defined above)alkyl, alkenyl, alkynyl, carbocyclyl, heterocyclyl and acyl groups. Alternatively two substituents together with the nitrogen to which they are attached may form a heterocyclyl group, preferably a 5 to 7-membered heterocyclyl group, which may be substituted and may contain other hetero atoms, for example morpholino, thiomorpholino or piperidinyl.

The term acyl includes the residues of sulphur and phosphorus-containing acids as well as carboxylic acids. Typically the residues are covered by the general formulae -C(=X^{a})R^{b}, -S(O)ₚR^{b} and -P(=X^{a})(OR^{a})(OR^{a}), where appropriate X^{a} is O or S, R^{b} is as defined for R^{a}, -OR^{a}, -SR^{a}, optionally substituted amino or acyl; and p is 1 or 2. Preferred groups are -C(=O)R^{c}, -C(=S)R^{c},and -S(O)ₚR^{c} where R^{c} is alkyl, C₁ to C₅ alkoxy, C₁ to C₅ alkylthio, phenyl, phenyloxy, phenylthio, carbocyclyl, heterocyclyl or amino, each of which may be substituted.

Complexes of compounds of the invention are usually formed from a salt of formula MAn or MAn₂, in which M is a metal cation, e.g. copper, manganese, cobalt, nickel, iron or zinc and An is an anion, e.g. chloride, nitrate or sulphate.

In cases where the compounds of the invention comprise a nitrogen atom which may be oxidised, N-oxides of such compounds are also part of the invention.

In cases where the compounds of the invention exist as the E and Z isomers, the invention includes individual isomers as well as mixtures thereof.

In cases where compounds of the invention exist as tautomeric isomers, the invention includes individual tautomers as well as mixtures thereof.

In cases where the compounds of the invention exist as optical isomers, the invention includes individual isomers as well as mixtures thereof, including the racemic mixture.

The compounds of the invention have activity as fungicides, especially against fungal diseases of plants, e.g. mildews and particularly cereal powdery mildew (*Erysiphe graminis*) and vine downy mildew (*Plasmopara viticola*), rice blast (*Pyricularia oryzae*), cereal eyespot (*Pseudocercosporella herpotrichoides*), rice sheath blight (*Pellicularia sasakii*), grey mould (*Botrytis cinerea*), damping off (*Rhizoctonia solani*), wheat brown rust (*Puccinia recondita*), late tomato or potato blight (*Phytophthora infestans*), apple scab (*Venturia inaequalis*), and glume blotch (*Leptosphaeria nodorum*). Other fungi against which the compounds may be active include other powdery mildews, other rusts, and other general pathogens of Deuteromycete, Ascomycete, Phycomycete and Basidomycete origin.

The invention thus also provides a method of combating fungi at a locus infested or liable to be infested therewith, which comprises applying to the locus a compound of formula I.

The invention also provides an agricultural composition comprising a compound of formula I in admixture with an agriculturally acceptable diluent or carrier.

The composition of the invention may of course include more than one compound of the invention.

In addition, the composition can comprise one or more additional active ingredients, for example compounds known to possess plant-growth regulant, herbicidal, fungicidal, insecticidal, acaricidal, antimicrobial or antibacterial properties. Alternatively the compound of the invention can be used in a simultaneous, sequential and/or alternative way with the other active ingredient(s).

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an *N*-acylsarcosinate; mono- or di-esters of phosphoric acid with fatty alcohol ethoxylates or alkyl phenol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and *N*-methyl taurine; the dialkyl sulphosuccinates, e.g. the sodium sulphonate of dioctyl succinate; acid derivatives of alkyl glycosides and alkylpolyglycosides materials and their metal salts, e.g. alkyl polyglycoside citrate or tartrate materials; or mono-, di- and tri-alkyl esters of citric acid and their metal salts.

Nonionic agents include condensation products of fatty acid esters, fatty alcohols, fatty acid amides or fatty-alkyl- or alkenyl-substituted phenols with ethylene and/or propylene oxide; fatty esters of polyhydric alcohol ethers, e.g. sorbitan fatty acid esters; condensation products of such esters with ethylene oxide, e.g. polyoxyethylene sorbitan fatty acid esters; alkyl glycosides, alkyl polyglycoside materials; block copolymers of ethylene oxide and propylene oxide; acetylenic glycols such as 2,4,7,9-tetramethyl-5-decyne-4,7-diol, ethoxylated acetylenic glycols; acrylic based graft copolymers; alkoxylated siloxane surfactants; or imidazoline type surfactants, e.g. 1-hydroxyethyl-2-alkylimidazoline.

Examples of a cationic surface-active agent include, for instance, an aliphatic mono-, di-, or polyamine as an acetate, naphthenate or oleate; an oxygen-containing amine such as an amine oxide, polyoxyethylene alkylamine or polyoxypropylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, an aerosol, a dispersion, an aqueous emulsion, a microemulsion, a dispersible concentrate, a dusting powder, a seed dressing, a fumigant, a smoke, a dispersible powder, an emulsifiable concentrate, granules or an impregnated strip. Moreover it can be in a suitable form for direct application or as a concentrate or primary composition which requires dilution with a suitable quantity of water or other diluent before application.

A dispersible concentrate comprises a compound of the invention dissolved in one or more water miscible or semi-water miscible solvents together with one or more surface active and/or polymeric material. Addition of the formulation to water results in the crystallisation of the active ingredient, the process being controlled by the surfactants and/or polymers resulting in a fine dispersion.

A dusting powder comprises a compound of the invention intimately mixed and ground with a solid pulverulent diluent, for example, kaolin.

An emulsifiable concentrate comprises a compound of the invention dissolved in a water-immiscible solvent which forms an emulsion or microemulsion on addition to water in the presence of an emulsifying agent.

A granular solid comprises a compound of the invention associated with similar diluents to those that may be employed in dusting powders, but the mixture is granulated by known methods. Alternatively it comprises the active ingredient absorbed or coated on a pre-formed granular carrier, for example, Fuller's earth, attapulgite, silica or limestone grit.

Wettable powders, granules or grains usually comprise the active ingredient in admixture with suitable surfactants and an inert powder diluent such as clay or diatomaceous earth.

Another suitable concentrate is a flowable suspension concentrate which is formed by grinding the compound with water or other liquid, surfactants and a suspending agent.

The concentration of the active ingredient in the composition of the present invention, as applied to plants is preferably within the range of 0.0001 to 1.0 per cent by weight, especially 0.0001 to 0.01 per cent by weight. In a primary composition, the amount of active ingredient can vary widely and can be, for example, from 5 to 95 per cent by weight of the composition.

In use a compound of the invention is generally applied to seeds, plants or their habitat. Thus, the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable application rate is within the range of from 5 to 1000 g per hectare, more preferably from 10 to 500 g per hectare.

Alternatively the active compound can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth, as this is the time when the plant can be most severely damaged. The spray or dust can conveniently contain a pre- or post-emergence herbicide if this is thought necessary. Sometimes, it is practicable to treat the roots, bulbs, tubers or other vegetative propagule of a plant before or during planting, for example, by dipping the roots in a suitable liquid or solid composition. When the active compound is applied directly to the plant a suitable rate of application is from 0.025 to 5 kg per hectare, preferably from 0.05 to 1 kg per hectare.

In addition, the compounds of the invention can be applied to harvested fruits, vegetables or seeds to prevent infection during storage.

In addition, the compounds of the invention can be applied to plants or parts thereof which have been genetically modified to exhibit a trait such as fungal and/or herbicidal resistance.

In addition the compounds of the invention can be used to treat fungal infestations in timber and in public health applications. Also the compounds of the invention can be used to treat fungal infestations in domestic and farm animals.

Compounds of the invention may be prepared, in known manner, in a variety of ways.

Compounds of general formula I may be prepared from compounds of general formula II which are reacted with R¹-CO-R² according to Scheme 1. Such reactant can be obtained from commercial suppliers or prepared by methods apparent to the skilled in the art. As a general manner, all starting materials used for the preparation of the compounds of the invention are either commercially available or can be prepared by well-known method from the skilled in the art. Such methods can for example be found in the literature, in patents, in the "Chemical Abstracts", in electronic databases or on the Internet.

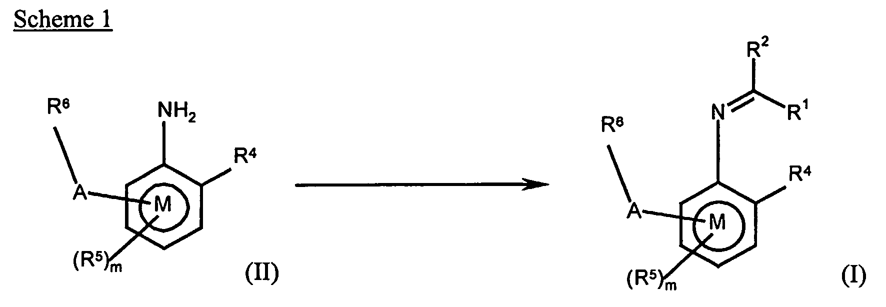

Such reaction is well known from the one skilled in the art and can be conducted according general references such as J. March, *Advanced Organic Chemistry,* IV edition, pages 1289 sqq.

Compounds of formula I can be thus prepared, for example, by combination of the following reactants listed in table A (compounds of formula II) and table B (compounds of formula R¹-CO-R²)below:

**- Table A -**

| | |
|---|---|
| 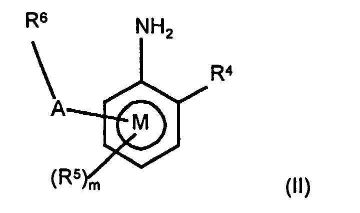 | |
| A1 | 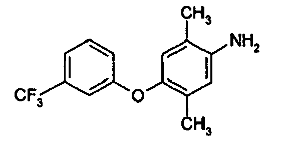 |
| A2 | 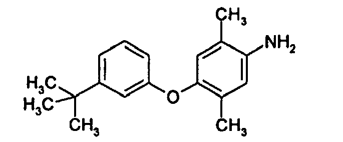 |
| A3 | 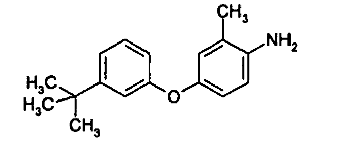 |
| A4 | 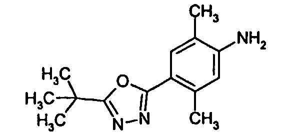 |
| A5 | 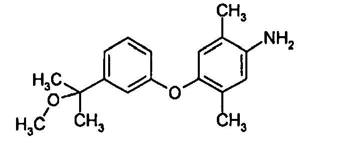 |
| A6 | 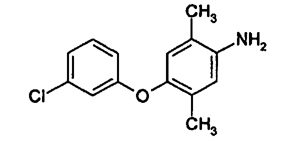 |
| A7 | 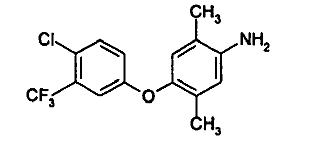 |
| A8 | 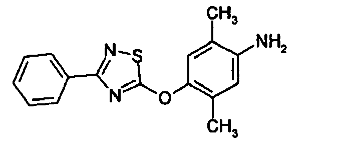 |
| A9 | 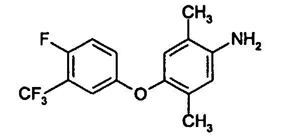 |
| A10 | 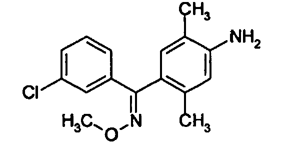 |

**- Table B -**

| | | |
|---|---|---|
| **R¹-CO-R²** | | |

| | **R¹** | **R²** |
|---|---|---|
| B1 | H | H |
| B2 | H | Cyclopropyl |
| B3 | H | 2-thiazolyl |
| B4 | H | 4-pyridinyl-N-oxide |
| B5 | H | Methylthioethyl |
| B6 | H | Trimethylammoniomethyl |
| B7 | H | 2-pyridyl |
| B8 | H | 1-methyl-2-pyrrolyl |
| B9 | H | Isopropyl |
| B10 | H | 5-nitro-2-furyl |
| B11 | H | 4-dimethylaminophenyl |
| B12 | H | Ethylaminoethyl |
| B13 | H | 2-methoxyvinyl |
| B14 | H | Trifluoromethyl |
| B15 | H | Phenyl |
| B16 | H | Methyl |
| B17 | Methyl | 2-pyridyl |
| B18 | Methyl | Ethylaminoethyl |
| B19 | Methyl | 2-methoxyvinyl |
| B20 | Methyl | Trifluoromethyl |
| B21 | Methyl | Cyclopropyl |
| B22 | Methyl | 2-thiazolyl |
| B23 | Methyl | 4-pyridinyl-N-oxide |
| B24 | Methyl | Methylthioethyl |
| B25 | Methyl | Trimethylammoniomethyl |
| B26 | Methyl | 1-methyl-2-pyrrolyl |
| B27 | Methyl | Isopropyl |
| B28 | Methyl | 5-nitro-2-furyl |
| B29 | Methyl | 4-dimethylaminophenyl |
| B30 | Methyl | Phenyl |
| B31 | Phenyl | Phenyl |
| B32 | Phenyl | 2-pyridyl |
| B33 | Phenyl | Ethylaminoethyl |
| B34 | Phenyl | 2-methoxyvinyl |
| B35 | Phenyl | Trifluoromethyl |
| B36 | Phenyl | Cyclopropyl |
| B37 | Phenyl | 2-thiazolyl |
| B38 | Phenyl | 4-pyridinyl-N-oxide |
| B39 | Phenyl | Methylthioethyl |
| B40 | Phenyl | Trimethylammoniomethyl |
| B41 | Phenyl | 1-methyl-2-pyrrolyl |
| B42 | Phenyl | Isopropyl |
| B43 | Phenyl | 5-nitro-2-furyl |
| B44 | Phenyl | 4-dimethylaminophenyl |

Compounds of general formula II may be prepared by reduction of the nitro group in compounds of formula III according to reaction scheme 2. Preferred reaction conditions comprise reaction with stannous chloride in concentrated hydrochloric acid.

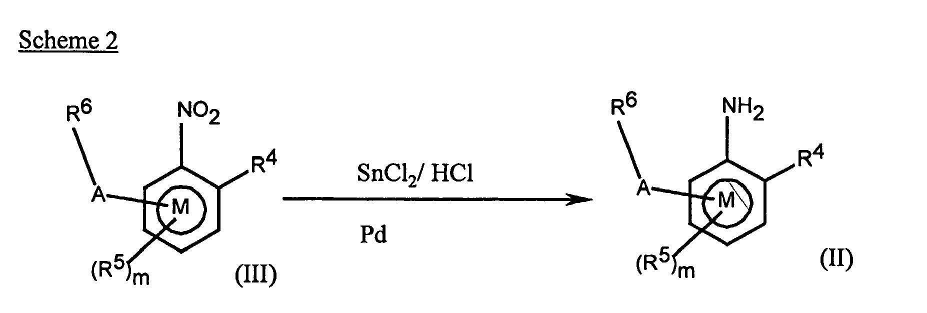

Compounds of formula IIa, i.e. compounds of general formula II where A is a direct bond, may be prepared according to reaction scheme 3, where X^{v} is a leaving group.

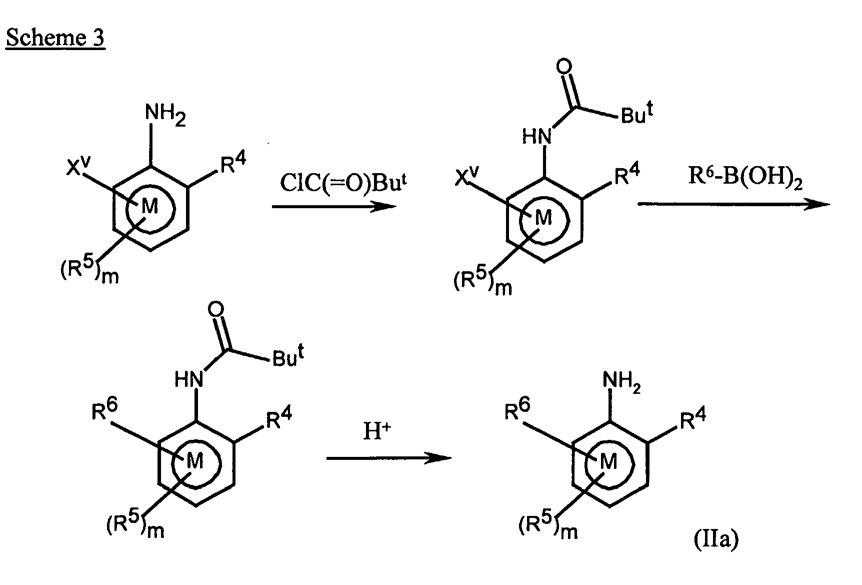

Compounds of formula IIb, i.e. compounds of general formula II where R⁴ is halogen, may be prepared according to scheme 4 where X^{T} represents halogen. When R⁴ is bromine preferred reaction conditions comprise stirring with bromine in a suitable solvent.

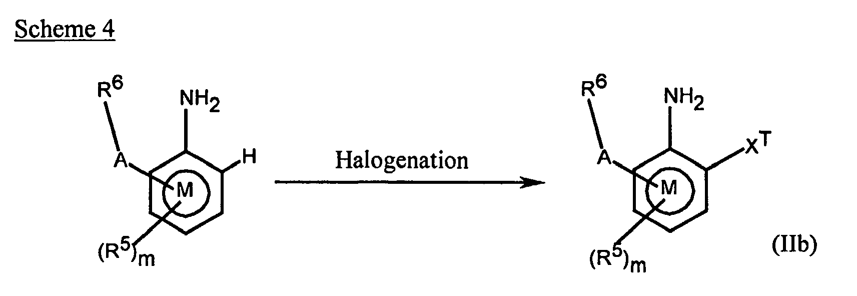

Compounds of formula IIc, i.e. compounds of general formula II where A is NHC(=O)-; compounds of formula IId, i.e. compounds of formula II where A is a direct bond and R⁶ is optionally substituted phthalimido, where the curved line connecting the 3 and 4 positions of the phthalimido group represents the optionally substituted carbocyclic ring; and compounds of formula IIe, i.e. compounds of general formula II where A is a direct bond and R⁶ is pyrrolyl, optionally substituted at the 2 and 5 positions by one or more groups R which may be the same or different; may be prepared from compounds of formula IV according to methodology shown in reaction scheme 5. For certain compounds of formula IV, protection/deprotection of the amino group ortho to R⁴ may be required to improve yields.

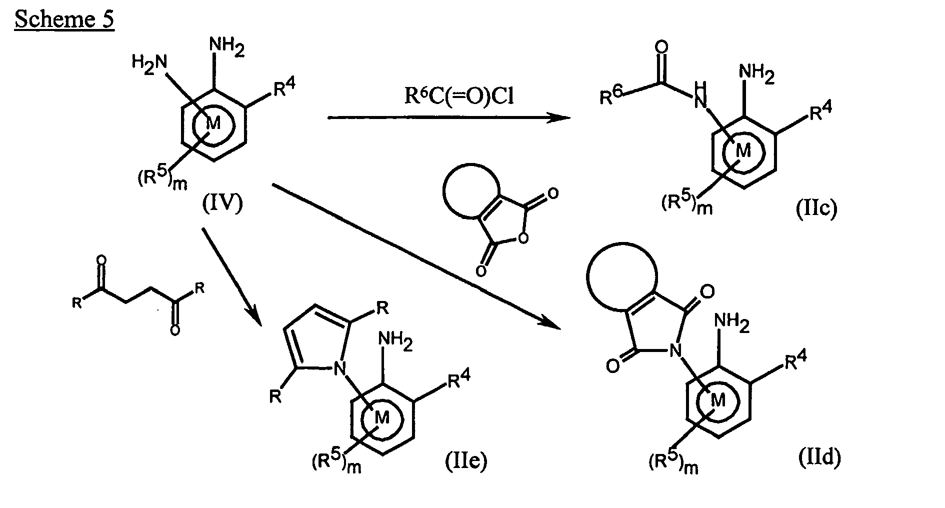

Compounds of formula IIIa, i.e. compounds of general formula III where A is a group A^{Z}, may be prepared by reacting compounds of formula V with compounds of formula VI according to reaction scheme 6. A^{Z} is a group which, in compound V, forms an anion under basic conditions. A^{Z} is alternatively a basic primary or secondary nitrogen atom. X^{Z} is a leaving group, preferably halogen. When A^{Z} is oxygen, preferred reaction conditions comprise treating V with sodium hydride followed by addition of VI. When A^{Z} is sulphur preferred reaction conditions comprise reacting V with VI in the presence of a tertiary amine base such as ethyldiisopropylamine. When A^{Z} is -CHR⁷-, preferred reaction conditions comprise treating V with potassium *tert*-butoxide in dimethylformamide at low temperature. When A^{Z} is a basic nitrogen atom, no base is required.

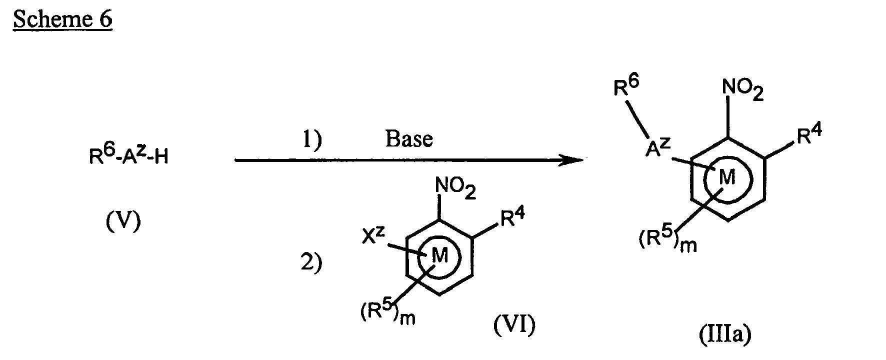

Compounds of formula IIIb, i.e. compounds of general formula III where A is a group A^{W}, may be prepared by reacting compounds of formula VII with compounds of formula VIII according to reaction scheme 7. A^{W} is a group which, in compound VII, forms an anion under basic conditions. X^{W} is a leaving group, preferably halogen. Preferred basic conditions comprise reaction of VII with potassium carbonate or sodium hydride followed by addition of VIII.

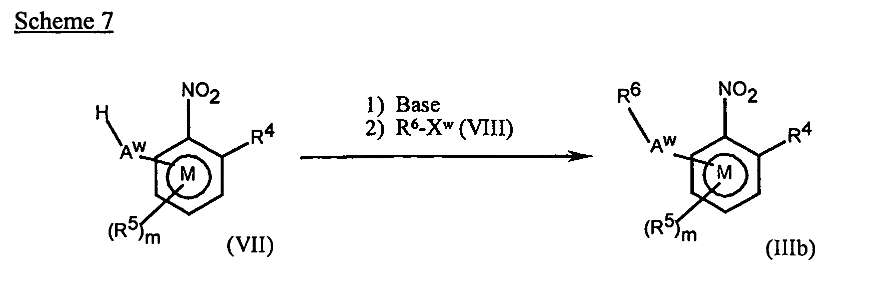

Compounds of formula IIIc, i.e. compounds of general formula III where A is O, may be prepared by reacting compounds of formula IX with boronic acids of formula X according to Scheme 8. Preferred reaction conditions comprise reaction with copper acetate and triethylamine.

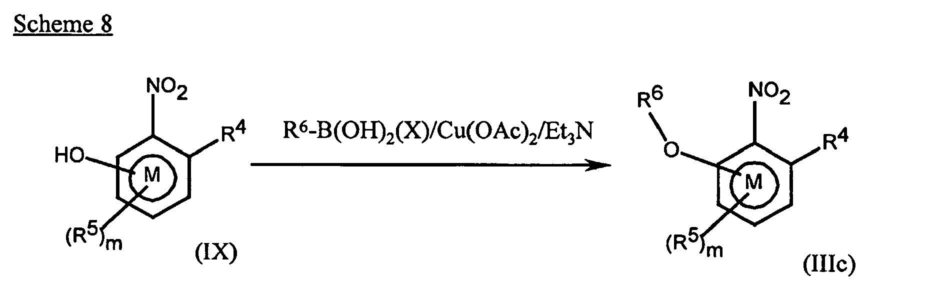

Compounds of formula IIId, i.e. compounds of formula III where A is a direct bond may be prepared according to reaction scheme 9 from compounds of formula XI where X^{Z} is a leaving group, preferably halogen.

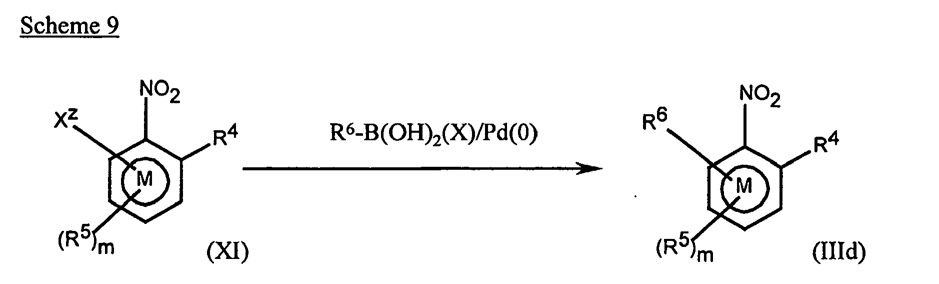

Compounds of formula III where A is a direct bond and R⁶ is a heterocyclyl can be prepared using a variety of methods known to a skilled chemist (for example see "Comprehensive Heterocyclic Chemistry", Vols 1-7, A. R. Katritzky and C. W. Rees). By way of example, routes to compounds of formula III containing a 1,2,4-oxadiazol-3-yl group (compound IIIe) and a 1,3,4-oxadiazol-2-yl group (compound IIIf) are shown in schemes 10 and 11.

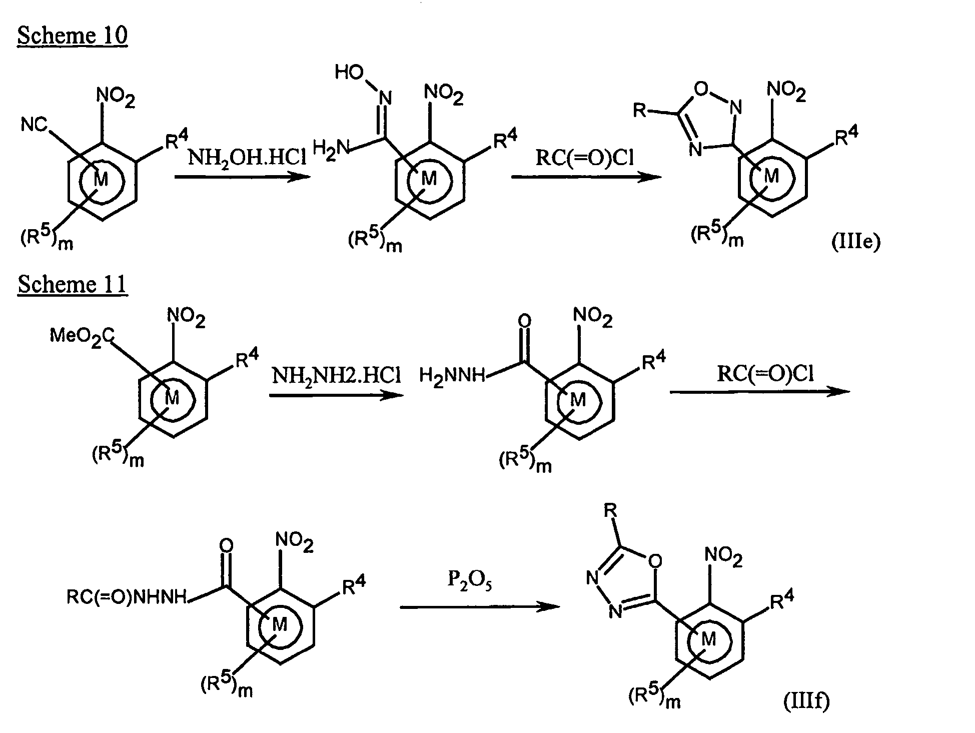

Alternatively, using similar chemistry to that described above, compounds of formula I can be prepared by introducing R⁶ after formation of the imine moiety.

Other methods will be apparent to the chemist skilled in the art, as will be methods for preparing starting materials and intermediates.

In addition, compounds of the invention may be prepared using combinatorial chemistry methodology.

The invention is illustrated in the following Examples. Structures of isolated, novel compounds were confirmed by N.M.R., mass spectrometry and/or other appropriate analyses. Proton N.M.R. spectra (¹H N.M.R.) were determined in deuterochloroform and chemical shifts (δ) are quoted in parts per million downfield of tetramethylsilane.

### Example 1: Preparation of compound 5 (see table 1 below)

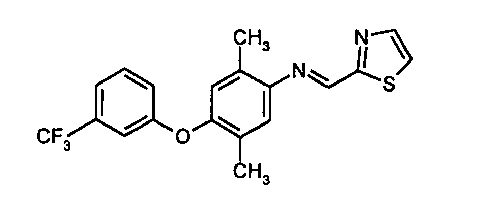

Step 1: Preparation of 2-nitro-5-(3-trifluoromethylphenoxy)-*p*-xylene
   To a suspension of sodium hydride (0.4 g of 60% in oil) in dry *N*-methylpyrrolidinone (10 ml) was slowly added 3-trifluoromethylphenol (1.62 g). When effervescence had ceased, 3-chloro-6-nitro-*p*-xylene (1.85 g) was added and the mixture stirred at 120-40°C for 5 hours. On cooling, the mixture was poured into water and the mixture extracted with diethyl ether (x3). The combined ether extracts were dried (MgSO₄), filtered and evaporated to give the title compound as a solid, m.p. 68-71°C.
Step 2: Preparation of 4-(3-trifluoromethylphenoxy)-2,5-xylidin (Compound A1, Table A)
   To a stirred mixture of stannous chloride (10.8 g) in concentrated hydrochloric acid (24 ml) and ethanol (50 ml) was added the product from Step 1 above (2.46 g) and the mixture was heated at 75°C for 2 hours. On cooling potassium hydroxide solution was added slowly with cooling. The mixture was extracted with diethyl ether (x3) and the combined extracts were washed with brine, dried (MgSO₄), filtered and evaporated to dryness to give a crude residue which was purified by silica gel chromatography eluting with light petroleum (b.p.60-80°C)/ethyl acetate (3:1) to give the title product, m.p. 58-60°C.
Step 3: Preparation of: 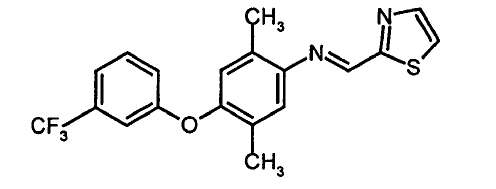 To the aniline A1 obtained from Step 2 (0.178g) and 2-thiazolylcarbaldehyde (compound B3, Table B) (0.0717g) dissolved in toluene (20ml) was added anhydrous magnesium sulphate (0.2g) and the reaction stirred at ambient temperature for 20 hours. The magnesium sulphate was filtered with toluene and the organic solvent was removed under reduced pressure to afford the title compound.
   *Mass spectroscopy analysis:* 377 (M+H).

### Example 2: Preparation of compound 16 (see table 1 below)

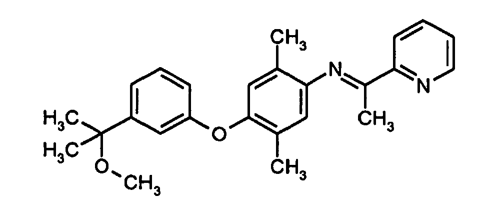

The aniline A5 (Table A) (0.18g) and the ketone B17 (Table B) (0.0758g) were refluxed for 20 hours in toluene (30ml) containing a catalytic amount of p-toluene sulphonic acid, collecting water by azeotropic distillation. The solution was cooled, washed with saturated sodium hydrogenocarbonate, dried over magnesium sulphate and solvent removed under reduced pressure. Purification by HPLC afforded the title compound.
*Mass spectroscopy analysis:* 389 (M+H).

The following compounds of formula Ia (see Table 1), i.e. compounds of general formula I where -A-R⁶ is para to the imine moiety, may be prepared by methods analogous to those of Examples 1 and 2.

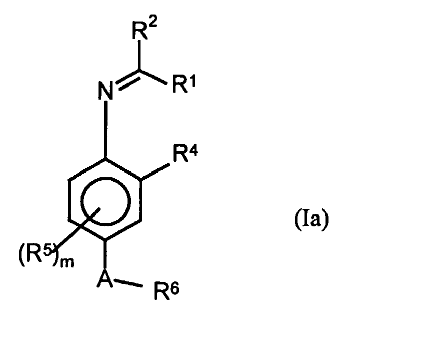

**Table 1**

| **Cmpd** | **R1** | **R2** | R4 | **R5** | A | **R6** | **MS result** |
|---|---|---|---|---|---|---|---|
| 1 | Phenyl | H | Me | 5-Me | O | 2-benzyloxyphenyl | |
| 2 | 2-thiazolyl | H | Me | 5-Me | O | 3-trifluoromethyl-4-chlorophenyl | 411 (M+H) |
| 3 | 2-thiazolyl | H | Me | 5-Me | O | 3-*t*-butylphenyl | 365 (M+H) |
| 4 | 2-thiazolyl | H | Me | 5-Me | O | 3-trifluoromethyl-4-fluorophenyl | 395 (M+H) |
| 5 | 2-thiazolyl | H | Me | 5-Me | O | 3-trifluoromethylphenyl | 377 (M+H) |
| 6 | 2-thiazolyl | H | Me | 5-Me | O | 3-chlorophenyl | 343 (M+H) |
| 7 | N-methyl-2-pyrrolyl | H | Me | 5-Me | O | 3-trifluoromethyl-4-chlorophenyl | 407 (M+H) |
| 8 | 2-nitro-5-furanyl | H | Me | 5-Me | O | 3-trifluoromethyl-4-fluorophenyl | 423 (M+H) |
| 9 | 2-nitro-5-furanyl | H | Me | 5-Me | O | 3-(1-methoxy-1-methylethyl)phenyl | 409 (M+H) |
| 10 | 4-dimethylaminophenyl | H | Me | 5-Me | O | 3-trifluoromethyl-4-fluorophenyl | 431 (M+H) |
| 11 | 2-nitro-5-furanyl | H | Me | 5-Me | O | 3-trifluoromethyl-4-Chlorophenyl | 439 (M+H) |
| 12 | 4-dimethylaminophenyl | H | Me | 5-Me | O | 3-trifluoromethyl-4-chlorophenyl | 447 (M+H) |
| 13 | 4-dimethylaminophenyl | H | Me | 5-Me | O | 3-*t*-butylphenyl | 401 (M+H) |
| 14 | N-methyl-2-pyrrolyl | H | Me | 5-Me | O | 3-(1-methoxy-1-methylethyl)phenyl | 377 (M+H) |
| 15 | N-methyl-2-pyrrolyl | H | Me | 5-Me | O | 3-trifluoromethylphenyl | 373 (M+H) |
| 16 | 2-pyridyl | Me | Me | 5-Me | O | 3-(1-methoxy-1-methylethyl)phenyl | 389 (M+H) |

### Test Examples

Compounds were assessed for activity against one or more of the following phytopathogenic diseases:
- *Erysiphe graminis f. sp. tritici*: wheat powdery mildew
- *Puccinia recondita*: wheat brown rust
- *Septoria nodorum*: wheat septoria nodorum
- *Septoria tritici*: wheat septoria tritici
- *Pyrenophora teres:* barley net blotch
Aqueous solutions or dispersions of the compounds of the invention at the desired concentration, including one or more wetting agents, were applied by spray or by drenching the stem base of the test plants, as appropriate. After a given time, plants or plant parts were inoculated with appropriate test pathogens and kept under controlled environmental conditions suitable for maintaining plant growth and development of the disease. After an appropriate time, the degree of infection of the affected part of the plant was visually estimated. At a concentration of 500 ppm (w/v) or less, the following compounds present a control of at least 65% on the specified fungal diseases versus non-treated test.
*Pyricularia grisea*: 2, 3, 4, 5, 6
*Pyricularia grisea*: 2, 3, 4, 5
*Fusarium culmorum*: 2
*Septoria tritici*: 4, 5
*Pythium ultimum*: 4

## Claims

1. A compound of general formula I and salts thereof 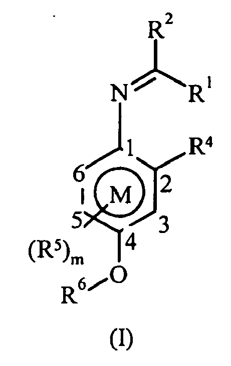 wherein
• R¹ is chosen from among alkyl, carbocyclyl, heterocyclyl, each of which may be substituted, and hydrogen;
• R² is chosen from among alkyl, carbocyclyl, each of which may be substituted, and hydrogen;
• R⁴ is chosen from among alkyl, which may be substituted; hydroxy; halogen;
• m is 0 to 3;
• when present R⁵, which may be the same or different to any other R⁵, is chosen from among alkyl, which may be substituted; hydroxy; halogen;
• R⁶ is optionally substituted carbo- or heterocyclyl; as well as E or Z- isomers and mixtures thereof; provided that
✔ R¹ cannot be selected in the list consisting of morpholinyl, pyrrolidinyl and piperidinyl while R² represents hydrogen, m is equal to 1, R⁴ and R⁵ represent methyl and R⁶ represents 3-trifluoromethylphenyl;
✔ R¹ does not represent piperidinyl while R² represents hydrogen, m is equal to 1, R⁴ and R⁵ represent methyl and R⁶ represents 3-t-butylphenyl;
✔ R¹ and R² cannot simultaneously represent hydrogen and 2-carbomethoxyphenyl while R⁴ represents hydroxy, m is equal to 0 and R⁶ represents 2-chloro-4-trifluoromethylphenyl.

2. A compound according to claim 1 wherein R¹ is alkyl which may be substituted by alkoxy, haloalkoxy, alkylthio, halogen or optionally substituted phenyl; or is hydrogen.

3. A compound according to claim 2 wherein R¹ is alkyl, , which may be substituted by alkoxy, haloalkoxy, alkylthio, halogen or phenyl optionally substituted by alkyl, haloalkyl, alkoxy, haloalkoxy or alkylthio, each containing 1 to 5 carbon atoms, or halogen, or is hydrogen.

4. A compound according to any preceding claim wherein R¹ is C₁-C₁₀ alkyl or hydrogen.

5. A compound according to any preceding claim wherein R¹ is methyl or hydrogen.

6. A compound according to any preceding claim wherein R² is alkyl, which may be substituted by alkoxy, haloalkoxy, alkylthio, halogen or optionally substituted phenyl, or is hydrogen.

7. A compound according to any preceding claim wherein R² is alkyl, which may be substituted by alkoxy, haloalkoxy, alkylthio, halogen or phenyl optionally substituted by alkyl, haloalkyl, alkoxy, haloalkoxy or alkylthio, each containing 1 to 5 carbon atoms, or by halogen, or is hydrogen.

8. A compound according to any preceding claim wherein R² is C₁-C₁₀ alkyl or hydrogen.

9. A compound according to any preceding claim wherein R² is methyl or hydrogen.

10. A compound according to any preceding claim wherein R⁴ is alkyl, which may be substituted by alkoxy, haloalkoxy, alkylthio, halogen or optionally substituted phenyl; or is hydroxy; halogen.

11. A compound according to any preceding claim wherein R⁴ is alkyl, which may be substituted by alkoxy, haloalkoxy, alkylthio, halogen or phenyl optionally substituted by alkyl, haloalkyl, alkoxy, haloalkoxy or alkylthio, each containing 1 to 5 carbon atoms, or halogen; or is hydroxy; halogen.

12. A compound according to any preceding claim wherein R⁴ is C₁-C₁₀ alkyl or halogen.

13. A compound according to any preceding claim wherein R⁴ is methyl or ethyl or halogen.

14. A compound according to any preceding claim wherein m is 0 or 1.

15. A compound according to any preceding claim wherein, when present, R⁵ is a group defined for R⁴ in either of claims 10 to 14.

16. A compound according to any preceding claim wherein, when present, R⁵ is attached at the 5 position of ring M.

17. A compound according to any preceding claim wherein A is -O-.

18. A compound according to any preceding claim wherein R⁶ is optionally substituted aromatic heterocyclyl.

19. A compound according to any preceding claim wherein R⁶ is optionally substituted thiazolyl, isothiazolyl, thiadiazolyl, pyridyl or pyrimidinyl.

20. A compound according to any preceding claim wherein R⁶ is optionally substituted 1,2,4-thiadiazolyl.

21. A compound according to any preceding claim wherein when substituted, R⁶ may be substituted by one or more substituents, which may be the same or different, and may be selected from the list: alkyl, alkenyl, alkynyl, carbo- or heterocyclyl, each of which may be substituted; hydroxy; mercapto; azido; nitro; halogen; cyano; acyl; optionally substituted amino; cyanato; thiocyanato; -SF₅; -OR^{a}; -SR^{a} and -Si(R^{a})₃, where R^{a} is alkyl, alkenyl, alkynyl, carbocyclyl or heterocyclyl, each of which may be substituted.

22. A compound according to claim 21 wherein when substituted, R⁶ may be substituted by one or more substituents, which may be the same or different, and may be selected from the list: hydroxy; halogen; cyano; acyl; amino; alkylamino; dialkylamino; alkyl; haloalkyl; R^{a}O-alkyl; acyloxyalkyl; cyano-oxyalkyl; alkoxy; haloalkoxy; alkylthio; carbocyclyl, optionally substituted by alkyl, haloalkyl, alkoxy, haloalkoxy or alkylthio; and benzyl optionally substituted by alkyl, haloalkyl, alkoxy, haloalkoxy or alkylthio.

23. The use of a compound according to any preceding claim and salts thereof as fungicides against fungal diseases of plants.

24. A fungicidal composition comprising at least one compound as claimed in any one of claims 1 to 23 in admixture with an agriculturally acceptable diluent or carrier.

25. A method of combating fungi of plants at a locus infested or liable to be infested therewith, which comprises applying to the locus a compound as claimed any one of claims 1 to 22.

## Patentansprüche

1. Verbindung der allgemeinen Formel I und Salze davon: 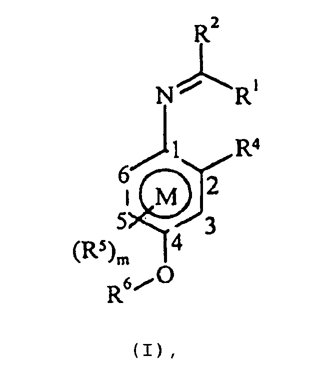 worin gilt:
- R¹ ist ausgewählt aus Alkyl, Carbocyclyl, Heterocyclyl, die alle substituiert sein können, und aus Wasserstoff;
- R² ist ausgewählt aus Alkyl, Carbocyclyl, die beide substituiert sein können, und aus Wasserstoff;
- R⁴ ist ausgewählt aus Alkyl, das substituiert sein kann, Hydroxy und aus Halogen;
- m ist 0 bis 3;
- liegt R⁵ vor, kann es, gleich oder verschieden voneinander, ausgewählt sein aus Alkyl, das substituiert sein kann, Hydroxy und aus Halogen;
- R⁶ ist gegebenenfalls substituiertes Carbo- oder Heterocyclyl; sowie E- oder Z-Isomere und Mischungen davon;
mit der Maßgabe, dass
- R¹ nicht aus der Liste ausgewählt sein kann, bestehend aus Morpholinyl, Pyrrolidinyl und Piperidinyl, wenn R² Wasserstoff, m 1, R⁴ und R⁵ Methyl und R⁶ 3-Trifluormethylphenyl darstellen;
- R¹ Piperidinyl nicht darstellt, wenn R² Wasserstoff, m 1, R⁴ und R⁵ Methyl und R⁶ 3-t-Butylphenyl darstellen;
- R¹ und R² Wasserstoff und 2-Carbomethoxyphenyl nicht gleichzeitig darstellen können, wenn R⁴ Hydroxy, m 0 und R⁶ 2-Chlor-4-trifluormethylphenyl darstellen.

2. Verbindung gemäß Anspruch 1, worin R¹ Alkyl, das mit Alkoxy, Haloalkoxy, Alkylthio, Halogen oder mit gegebenenfalls substituiertem Phenyl substituiert sein kann; oder Wasserstoff ist.

3. Verbindung gemäß Anspruch 2, worin R¹ Alkyl, das mit Alkoxy, Haloalkoxy, Alkylthio, Halogen oder mit Phenyl substituiert sein kann, das gegebenenfalls mit Alkyl, Haloalkyl, Alkoxy, Haloalkoxy oder Alkylthio, die jeweils 1 bis 5 Kohlenstoffatome enthalten, oder mit Halogen substituiert ist, oder Wasserstoff ist.

4. Verbindung gemäß einem der vorhergehenden Ansprüche, worin R¹ C₁₋₁₀-Alkyl oder Wasserstoff ist.

5. Verbindung gemäß einem der vorhergehenden Ansprüche, worin R¹ Methyl oder Wasserstoff ist.

6. Verbindung gemäß einem der vorhergehenden Ansprüche, worin R² Alkyl, das mit Alkoxy, Haloalkoxy, Alkylthio, Halogen oder mit gegebenenfalls substituiertem Phenyl substituiert sein kann, oder Wasserstoff ist.

7. Verbindung gemäß einem der vorhergehenden Ansprüche, worin R² Alkyl, das mit Alkoxy, Haloalkoxy, Alkylthio, Halogen oder mit Phenyl substituiert sein kann, das gegebenenfalls mit Alkyl, Haloalkyl, Alkoxy, Haloalkoxy oder mit Alkylthio, die jeweils 1 bis 5 Kohlenstoffatome enthalten, oder mit Halogen substituiert ist, oder Wasserstoff ist.

8. Verbindung gemäß einem der vorhergehenden Ansprüche, worin R² C₁₋₁₀-Alkyl oder Wasserstoff ist.

9. Verbindung gemäß einem der vorhergehenden Ansprüche, worin R² Methyl oder Wasserstoff ist.

10. Verbindung gemäß einem der vorhergehenden Ansprüche, worin R⁴ Alkyl, das mit Alkoxy, Haloalkoxy, Alkylthio, Halogen oder mit gegebenenfalls substituiertem Phenyl substituiert sein kann, Hydroxy oder Halogen ist.

11. Verbindung gemäß einem der vorhergehenden Ansprüche, worin R⁴ Alkyl, das mit Alkoxy, Haloalkoxy, Alkylthio, Halogen oder mit Phenyl substituiert sein kann, das gegebenenfalls mit Alkyl, Haloalkyl, Alkoxy, Haloalkoxy oder Alkylthio, die jeweils 1 bis 5 Kohlenstoffatome enthalten, oder mit Halogen substituiert ist, Hydroxy oder Halogen ist.

12. Verbindung gemäß einem der vorhergehenden Ansprüche, worin R⁴ C₁₋₁₀-Alkyl oder Halogen ist.

13. Verbindung gemäß einem der vorhergehenden Ansprüche, worin R⁴ Methyl oder Ethyl oder Halogen ist.

14. Verbindung gemäß einem der vorhergehenden Ansprüche, worin m 0 oder 1 ist.

15. Verbindung gemäß einem der vorhergehenden Ansprüche, worin, bei Vorliegen, R⁵ eine für R⁴ in jedem der Ansprüche 10 bis 14 definierte Gruppe ist.

16. Verbindung gemäß einem der vorhergehenden Ansprüche, worin, bei Vorliegen, R⁵ an die 5-Position des Rings M gebunden ist.

17. Verbindung gemäß einem der vorhergehenden Ansprüche, worin A -O- ist.

18. Verbindung gemäß einem der vorhergehenden Ansprüche, worin R⁶ gegebenenfalls substituiertes aromatisches Heterocyclyl ist.

19. Verbindung gemäß einem der vorhergehenden Ansprüche, worin R⁶ gegebenenfalls substituiertes Thiazolyl, Isothiazolyl, Thiadiazolyl, Pyridyl oder Pyrimidinyl ist.

20. Verbindung gemäß einem der vorhergehenden Ansprüche, worin R⁶ gegebenenfalls substituiertes 1,2,4-Thiadiazolyl ist.

21. Verbindung gemäß einem der vorhergehenden Ansprüche, worin, bei Substitution, R⁶ substituiert sein kann mit einem oder mehreren Substituenten, die, gleich oder verschieden, ausgewählt sein können aus der Liste: Alkyl, Alkenyl, Alkinyl, Carbo- oder Heterocyclyl, die jeweils substituiert sein können; Hydroxy; Mercapto; Azido; Nitro; Halogen; Cyano; Acyl; gegebenenfalls substituiertes Amino; Cyanato; Thiocyanato; -SF₅; -OR^{a}; -SR^{a} und -Si(R^{a})₃, worin R^{a} Alkyl, Alkenyl, Alkinyl, Carbocyclyl oder Heterocyclyl ist, die jeweils substituiert sein können.

22. Verbindung gemäß Anspruch 21, worin, bei Substitution, R⁶ mit einem oder mehreren Substituenten substituiert sein kann, die, gleich oder verschieden, ausgewählt sein können aus der Liste: Hydroxy; Halogen; Cyano; Acyl; Amino; Alkylamino; Dialkylamino; Alkyl; Haloalkyl; R^{a}O-Alkyl; Acyloxyalkyl; Cyanooxyalkyl; Alkoxy; Haloalkoxy; Alkylthio; Carbocyclyl, gegebenenfalls substituiert mit Alkyl, Haloalkyl, Alkoxy, Haloalkoxy oder mit Alkylthio; und Benzyl, gegebenenfalls substituiert mit Alkyl, Haloalkyl, Alkoxy, Haloalkoxy oder mit Alkylthio.

23. Verwendung einer Verbindung gemäß einem der vorhergehenden Ansprüche oder von Salzen davon als Fungizide gegen fungale Pflanzenkrankheiten.

24. Fungizide Zusammensetzung, umfassend mindestens eine Verbindung gemäß einem der Ansprüche 1 bis 23 in Abmischung mit landwirtschaftlich geeigneten Verdünnungsmitteln oder Trägern.

25. Verfahren zur Bekämpfung von Fungi von Pflanzen am damit geschädigten oder gefährdeten Ort, wobei man an dem Ort eine Verbindung gemäß einem der Ansprüche 1 bis 22 anwendet und aufbringt.

## Revendications

1. Composé de formule générale I et ses sels 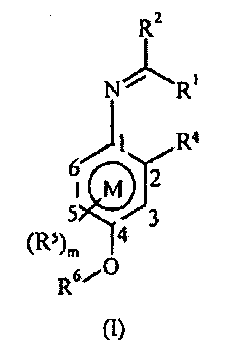 dans laquelle
• R¹ est choisi entre des groupes alkyle, carbocyclyle et hétérocyclyle, chacun pouvant être substitué, et un atome d'hydrogène ;
• R² est choisi entre des groupes alkyle, et carbocyclyle, chacun pouvant être substitué, et un atome d'hydrogène ;
• R⁴ est choisi entre des groupes alkyle, qui peuvent être substitués, hydroxy et halogéno ;
• m a une valeur de 0 à 3 ;
• lorsqu'il est présent, R⁵, qui peut être identique à, ou différent de, n'importe quel autre groupe R⁵, est choisi entre des groupes alkyle, qui peuvent être substitués, hydroxy et halogéno ;
• R⁶ représente un groupe carbo- ou hétérocyclyle facultativement substitué ; ainsi que leurs isomères E ou Z et leurs mélanges ; sous réserve que :
✔ R¹ ne puisse être choisi dans la liste consistant en des groupes morpholinyle, pyrrolidinyle et pipéridinyle tandis que R² représente un atome d'hydrogène, m est égal à 1, R⁴ et R⁵ représentent des groupes méthyle et R⁶ représente un groupe 3-trifluoro-méthylphényle ;
✔ R¹ ne représente pas un groupe pipéridinyle tandis que R² représente un atome d'hydrogène, m est égal à 1, R⁴ et R⁵ représentent des groupes méthyle et R⁶ représente un groupe 3-tertiobutylphényle ;
✔ R¹ et R² ne puissent représenter simultanément un atome d'hydrogène et un groupe 2-carbométhoxyphényle tandis que R⁴ représente un groupe hydroxy, m est égal à 0 et R⁶ représente un groupe 2-chloro-4-trifluorométhylphényle.

2. Composé suivant la revendication 1, dans lequel R¹ représente un groupe alkyle qui peut être substitué avec un substituant alkoxy, halogénalkoxy, alkylthio, halogéno ou un groupe phényle facultativement substitué ; ou bien représente un atome d'hydrogène.

3. Composé suivant la revendication 2, dans lequel R¹ représente un groupe alkyle qui peut être substitué avec un groupe alkoxy, halogénalkoxy, alkylthio, halogéno, ou un groupe phényle facultativement substitué avec un groupe alkyle, halogénalkyle, alkoxy, halogénalkoxy ou alkylthio, chacun contenant 1 à 5 atomes de carbone, ou un atome d'halogène, ou bien représente un atome d'hydrogène.

4. Composé suivant l'une quelconque des revendications précédentes, dans lequel R¹ représente un groupe alkyle en C₁ à C₁₀ ou un atome d'hydrogène.

5. Composé suivant l'une quelconque des revendications précédentes, dans lequel R¹ représente un groupe méthyle ou un atome d'hydrogène.

6. Composé suivant l'une quelconque des revendications précédentes, dans lequel R² représente un groupe alkyle qui peut être substitué avec un groupe alkoxy, halogénalkoxy, alkylthio, halogéno ou un groupe phényle facultativement substitué ; ou bien représente un atome d'hydrogène.

7. Composé suivant l'une quelconque des revendications précédentes, dans lequel R² représente un groupe alkyle, qui peut être substitué avec un groupe alkoxy, halogénalkoxy, alkylthio, halogéno, ou un groupe phényle facultativement substitué avec un groupe alkyle, halogénalkyle, alkoxy, halogénalkoxy ou alkylthio, chacun contenant 1 à 5 atomes de carbone, ou avec un atome d'halogène, ou bien représente un atome d'hydrogène.

8. Composé suivant l'une quelconque des revendications précédentes, dans lequel R² représente un groupe alkyle en C₁ à C₁₀ ou un atome d'hydrogène.

9. Composé suivant l'une quelconque des revendications précédentes, dans lequel R² représente un groupe méthyle ou un atome d'hydrogène.

10. Composé suivant l'une quelconque des revendications précédentes, dans lequel R⁴ représente un groupe alkyle, qui peut être substitué avec un groupe alkoxy, halogénalkoxy, alkylthio, halogéno ou un groupe phényle facultativement substitué ; ou représente un groupe hydroxy ; ou un atome d'halogène.

11. Composé suivant l'une quelconque des revendications précédentes, dans lequel R⁴ représente un groupe alkyle, qui peut être substitué avec un groupe alkoxy, halogénalkoxy, alkylthio, halogéno, ou un groupe phényle facultativement substitué avec un groupe alkyle, halogénalkyle, alkoxy, halogénalkoxy ou alkylthio, chacun contenant 1 à 5 atomes de carbone, ou un atome d'halogène, ou bien représente un groupe hydroxy ; ou un atome d'halogène.

12. Composé suivant l'une quelconque des revendications précédentes, dans lequel R⁴ représente un groupe alkyle en C₁ à C₁₀ ou un atome d'halogène.

13. Composé suivant l'une quelconque des revendications précédentes, dans lequel R⁴ représente un groupe méthyle ou éthyle ou un atome d'halogène.

14. Composé suivant l'une quelconque des revendications précédentes, dans lequel m est égal à 0 ou 1.

15. Composé suivant l'une quelconque des revendications précédentes, dans lequel, lorsqu'il est présent, R⁵ représente un groupe défini pour R⁴ dans l'une quelconque des revendications 10 à 14.

16. Composé suivant l'une quelconque des revendications précédentes, dans lequel, lorsqu'il est présent, R⁵ est fixé en position 5 du noyau M.

17. Composé suivant l'une quelconque des revendications précédentes, dans lequel A représente un groupe -O-.

18. Composé suivant l'une quelconque des revendications précédentes, dans lequel R⁶ représente un groupe hétérocyclyle aromatique facultativement substitué.

19. Composé suivant l'une quelconque des revendications précédentes, dans lequel R⁶ représente un groupe thiazolyle, isothiazolyle, thiadiazolyle, pyridyle ou pyrimidinyle facultativement substitué.

20. Composé suivant l'une quelconque des revendications précédentes, dans lequel R⁶ représente un groupe 1,2,4-thiadiazolyle facultativement substitué.

21. Composé suivant l'une quelconque des revendications précédentes, dans lequel, lorsqu'il est substitué, R⁶ peut être substitué avec un ou plusieurs substituants, qui peuvent être identiques ou différents, et qui peuvent être choisis dans la liste comprenant : des substituants alkyle, alcényle, alcynyle, carbo- ou hétérocyclyle, chacun pouvant être substitué ; hydroxy ; mercapto ; azido ; nitro ; halogéno ; cyano ; acyle ; amino facultativement substitué ; cyanato ; thiocyanato ; -SF₅ ; -OR^{a} ; -SR^{a} et -Si(R^{a})₃, dans lesquels R^{a} représente un groupe alkyle, alcényle, alcynyle, carbocyclyle ou hétérocyclyle, chacun pouvant être substitué.

22. Composé suivant la revendication 21, dans lequel, lorsqu'il est substitué, R⁶ peut être substitué avec un ou plusieurs substituants, qui peuvent être identiques ou différents, et qui peuvent être choisis dans la liste comprenant : des substituants hydroxy ; halogéno ; cyano ; acyle ; amino ; alkylamino ; dialkylamino ; alkyle ; halogénalkyle ; R^{a}O-alkyle acyloxyalkyle ; cyano-oxyalkyle alkoxy ; halogénalkoxy ; alkylthio ; carbocyclyle, facultativement substitué avec un groupe alkyle, halogénalkyle, alkoxy, halogénalkoxy ou alkylthio ; et benzyle facultativement substitué avec un groupe alkyle, halogénalkyle, alkoxy, halogénalkoxy ou alkylthio.

23. Utilisation d'un composé suivant l'une quelconque des revendications précédentes et de ses sels comme fongicide contres des maladies fongiques de végétaux.

24. Composition fongicide comprenant au moins un composé suivant l'une quelconque des revendications 1 à 23 en mélange avec un diluant ou support acceptable en agriculture.

25. Procédé pour lutter contre des champignons de végétaux dans un milieu infesté ou susceptible d'être infesté par ces champignons, qui comprend l'application à ce milieu d'un composé suivant l'une quelconque des revendications 1 à 22.
